Europäisches Patentamt

(19) European Patent Office

Office européen des brevets

(11) Publication number: **0 250 119**
**A2**

## EUROPEAN PATENT APPLICATION

(21) Application number: **87304850.8**

(22) Date of filing: **02.06.87**

(51) Int. Cl.⁴: **G01N 33/53** , G01N 33/543 , C07K 15/00 , C12P 21/00 , //G01N33/564,G01N33/68,G01-N33/577,(C12P21/00,C12R1:91)

(30) Priority: **20.06.86 US 876741**

(43) Date of publication of application:
**23.12.87 Bulletin 87/52**

(84) Designated Contracting States:
**AT BE CH DE ES FR GB GR IT LI LU NL SE**

(71) Applicant: **VASOCOR**
**2438 Wyandotte Street**
**Mountain View California 94013(US)**

(72) Inventor: **Calenoff, Emanuel**
**1824 Oak Creek Drive No. 319**
**Palo Alto California 94304(US)**

(74) Representative: **Harrison, David Christopher et al**
**MEWBURN ELLIS & CO 2/3 Cursitor Street**
**London EC4A 1BQ(GB)**

(54) **Atherosclerotic anti-idiotype antibody immunoassay and reagents.**

(57) The method of this invention for determining the presence of atherosclerotic plaque antigen in serum comprises contacting the serum with anti-plaque antibody for a time sufficient to permit binding of the atherosclerotic plaque antigen with the anti-plaque antibody, and determining the atherosclerotic anti-idotype antibody, if any binding with the anti-plaque antibody. The anti-plaque antibody can be bound to an insoluble support, contacted with serum to permit atherosclerotic anti-idiotype antibody conjugation with the anti-plaque antibody, and the amount of resultant atherosclerotic anti-idiotype antibody bound to the insoluble support can be determined.

Alternatively, a precise amount of atherosclerotic anti-idiotype antibody can be bound to the insoluble support, and a mixture of a predetermined excess amount of labeled anti-plaque antibody and serum can be contacted therewith. The labeled anti-plaque antibody bound to the insoluble support or remaining in the mixture can be determined. Also, a precise amount of anti-plaque antibody can be bound to the insoluble support, and a mixture of a predetermined amount of labeled atherosclerotic anti-idiotype antibody and serum contacted therewith. The labeled atherosclerotic anti-idiotype antibody bound to the insoluble support or remaining in the solution can then be determined.

# ATHEROSCLEROTIC ANTI-IDIOTYPE ANTIBODY IMMUNOASSAY AND REAGENTS

This invention relates to an immunoassay method and reagents for determining the presence of atherosclerotic plaque. In particular, this invention is a method and reagents for determining in patient serum the presence of anti-idiotype antibodies which bind specifically to the variable region or binding sites of antibodies which bind preferentially with atherosclerotic plaque antigen. These are denoted hereinafter as atherosclerotic anti-idiotype antibodies or simply as anti-idiotype antibodies..

The endothelium located between the blood and arterial tissue serves as a barrier against the accumulation of blood components in the vascular wall. Formation of atherosclerotic lesions in the sub-endothelium is associated with major coronary disease and stroke, and the causes and detection of such lesions has been intensely investigated.

Endothelial injury, believed to be an initial step in the formation of the atherosclerotic lesions, can be potentially caused by hemodynamic strain, hypercholesterolemia, hypertension and immune complex disease. Endothelial injury leads to intimal thickening, cell proliferation, cholesterol accumulation and formation of connective tissue fibers. IgG and complement factor C3 accumulation in injured endothelial cells and nonendothehelialized intima has been observed. Mononuclear phagocytes derived from blood have also been found to constitute a part of the cell population in atherosclerotic lesions. The mechanisms leading to the ultimate plaque composition are as yet unknown.

A variety of soluble proteins have been extracted from human atherosclerotic plaque, including IgA, IgG, IgM, B1C(C3), alpha$_1$-antitrypsin, alpha$_2$-macroglobulin, fibrinogen, albumin, LDL, HDL, alpha$_1$-acid glycoprotein, $\beta_2$-glycoprotein, transferrin and ceruloplasmin. The diseased intima was also found to contain a small amount of tissue-bound IgG, IgA and B1C. Hollander, W. et al, Atherosclerosis. 34:391-405 (1979). Other scientists have reported IgG in the lesions and adjacent endothelial tissue. Parums, D. et al, Atherosclerosis. 38:211-216 (1981), Hansson, G. et al, Experimental and Molecular Pathology. 34:264-280 (1981), Hansson, G. et al, Acta Path. Microbiol. Immunol. Scand. Sect.A. 92:429-435 (1984). However, the origin, function and binding properties of the immunoglobulins in the atherosclerotic and associated tissue is still a mystery. Anti-low density lipoprotein (LDL) autoantibodies has been reported to be higher in patients of vascular disease, suggesting that they are associated in some way with arteriosclerotic manifestations. However, no causal relationship between these autoantibodies and arteriosclerotic plaque has been established. Szondy, E. et al, Mechanisms of Aging and Development. 29:117-123 (1985).

In my copending application Serial No. 846,401 filed March 31, 1986, I have described a method for determining the presence of atherosclerotic plaque in a patient by determining the presence of antibodies which bind to the atherosclerotic plaque (hereinafter denoted as anti-plaque antibodies). In the immunoassay, the antibodies were separated from the serum by conjugating them with insolubilized plaque.antigen. Surprisingly, I found that in the serum of some patients with most advanced atherosclerosis, the anti-plaque antibodies would not bind to the insolubilized antibody. As a result of the immunoregulation system, it appears that the level of anti-plaque antibodies with available binding sites in the serum at certain times can be lower than at other times, corresponding to the increase of in serum level of atherosclerotic anti-idiotype antibodies which bind specifically to the variable region or binding sites of the anti-plaque antibodies. I have found that measurement of both the circulating anti-plaque antibody and the anti-idiotype antibodies is necessary in advanced cases to determine the severity or stage of progression of the atherosclerosis.

It is an object of this invention to determine the presence of atherosclerotic anti-idiotype antibodies in patient serum as an indication of the presence of advanced atherosclerosis.

A wide variety of immunoassays have been developed for determining the presence and amount of a wide variety of antigenic and non-antigenic materials in diverse body fluids and tissues. Total immunoglobulin and IgE immunoassays are described in U.S. patents 3,720,760 and 4,444,879. IgG allotype immunoassays are described in Russian Patent 649,433. ELISA immunoassays are described by Maggio, et al, ENZYME-IMMUNOASSAY. Boca Raton: CRC Press pp 172-176 (1980).

A method for detecting anti-idiotype antibodies in serum by an inhibition immunoassay for the diagnosis of small cell lung cancer is described in U.S. 6,717,692 (Derwent WPI Abstract No. 85-310117/49 corresponding to United States Application No. 717,692 filed March 29, 1985). The preparation of anti-idiotype antibodies and their use in immunoassays is described in U.S. Patents 4,513,088 and 4,513,088.

Preparation of anti-idiotype antibodies from peptides characteristic of antibody variable regions is described by Chen, P. et al, J.Exp.Med. 162:487-500 (1985), and in PTC Application No. 8502909 and EPA Application No. 141783. The preparation of monoclonal anti-idiotype antibodies to tumor antigen antibodies from tumor antigen is described in U.S. Patent 4,513,088.

The method of this invention comprises contacting serum with anti-plaque antibody for a time sufficient to permit anti-plaque antibody binding with atherosclerotic anti-idiotype antibody to occur and determining the extend of such binding.

One embodiment of this method comprises

a) contacting a sample with an insoluble support to which non-human anti-plaque antibody is adhered for a time sufficient to permit antigen-antibody binding to occur and removing unconjugated material from the support;

b) contacting the insoluble support with labeled anti-human Ig antibody for a time sufficient to permit antigen-antibody binding to occur and removing unconjugated material from the support, and

c) determining the label adhering to the insoluble support.

Another embodiment of this method comprises

a) contacting an insoluble support to which anti-plaque antibody is adhered with a solution of sample and a non-excess amount of labeled atherosclerotic anti-idiotype antibody for a time sufficient to permit antigen-antibody binding to occur and removing unconjugated material from the support;

b) determining the label adhering to the insoluble support or remaining in the solution.

Still another embodiment of this method comprises

a) contacting an insoluble support to which atherosclerotic anti-idiotype antibody is adhered with a solution of sample and a non-excess amount of labeled atherosclerotic anti-plaque antibody for a time sufficient to permit antigen-antibody binding to occur and removing unconjugated material from the support;

b) determining the label adhering to the insoluble support or remaining in the solution.

Insoluble supports to which atherosclerotic anti-idiotype antibodies are adhered and labeled atherosclerotic anti-idiotype antibodies are reagents of this invention.

The different embodiments of the method of this invention have the common steps of contacting serum with anti-plaque antibody for a time sufficient to permit anti-plaque antibody binding with atherosclerotic anti-idiotype antibody to occur and determining the extend of such binding.

The anti-plaque antibody can be derived directly from atherosclerotic plaque or preferably from purified atherosclerotic plaque antigen by conventional antiserum or monoclonal techniques. Plaque antigen is obtained by processing human arteriosclerotic plaque to isolate and purify antigen specific thereto. The plaque can be obtained by processing human aortas from patients who have died from myocardial infarction, cerebral vascular accident or can be obtained from surgical procedures. The aortae are dissected free of surrounding tissue and fat and rinsed in saline to remove contaminating blood. Segments of plaques are separated and processed.

The plaque areas are removed, cut into small pieces, washed briefly, and homogenized in isotonic solution such as phosphate-buffered saline (PBS). The homogenate repeatedly centrifuged and resuspended in PBS, the eluates being collected. The sedimented debris can also be extracted with citrate buffer, acid pH, and the eluate is neutralized and purified by dialysis. The supernatant as well as the pooled PBS eluates can be concentrated by vacuum dialysis and ultrafiltration to yield a concentrate of soluble plaque antigens.

Insoluble plaque antigens can be obtained by digesting portions with different enzymes, extracting the digest with PBS, and concentrating the eluates as described above. The enzyme compositions used can include collagenase, elastase, DNase, hyaluronidase, and neuraminidase. Each digest is centrifuged, concentrated as by ultrafiltration, for example, and dialyzed to yield a concentrated solution of insoluble plaque antigens.

Polyclonal anti-plaque antibody can be obtained by immunizing an animal such as a rabbit, guinea pig, rat or goat with concentrated atherosclerotic plaque antigen, removing serum from the immunized animal, and separating the immunoglobulins from the serum, for example by ammonium sulfate precipitation. The principal antibodies useful in the method of this invention are IgG and IgM antibodies, although the IgE and IgA antibodies can also be used if available in sufficient quantity.

Monoclonal anti-plaque antibody can be obtained from hybridized spleen cells of mice immunized with purified plaque antigen by the methods of Glafre and Milstein, Methods of Enzym. 73:1 (1981).

Polyclonal anti-idiotype antibodies can be prepared by passing the animal serum obtained by immunizing with atherosclerotic plaque antigen through a column to which anti-plaque antibody has been covalently bonded by conventional procedures well known in the art. After rinsing to remove residual serum, the column is eluted with a suitable buffer such as sodium acetate buffer to remove the anti-idiotype antibody which has been separated, and the eluate is purified by dialysis to remove small molecules in a conventional manner.

3

Monoclonal anti-idiotype antibodies can be prepared by screening the clones from the hybridized spleen cells of mice immunized with purified plaque antigen or with anti-plaque antibodies the methods of Glafre and Milstein, Methods of Enzym. (supra) for anti-idiotype antibodies using a method of this invention. Anti-plaque antibody bound to a microwell is incubated with hybridoma supernatant for a time sufficient to permit antibody-antigen binding to occur, the supernatant is removed from the well, and the well is rinsed. The well is then incubated with enzyme labeled goat or rabbit anti-Ig for a time sufficient to permit antibody-antigen binding to occur and the residue is removed from the well by rinsing. A substrate yielding a chromophore or fluorophore in the presence of the enzyme is then added to the well, and the presence the chromophore or fluorophore is used as a criterion for selecting clones.

In the preferred procedures according to this invention, an insoluble support to which either anti-plaque antibody or anti-idiotype antibody is adhered is contacted (incubated) with the sample or a mixture of the sample and further reagent. The anti-plaque antibody and anti-idiotype antibody can be adhered or bonded to the insoluble support by conventional procedures well known in the art. Procedures for binding of antibodies to insoluble supports are described in U.S. patents 3,551,555, 3,553,310, 4,048,298 and RE-29,474, for example. Binding of antibodies to polystyrene by adsorption has been described in U.S. patents 3,646,346 and 4,092,408, for example. Binding of protein containing antigens to a variety of insoluble supports has been described in U.S. Patent 3,720,760.

A variety of materials can be used as the insoluble support, the primary consideration being the binding of the anti-plaque antibody or the plaque antigen to the surface, the absence of interference with the anti-plaque antibody and plaque antigen conjugating reaction or with other reactions which can be employed to determine the presence and extent of the conjugating reaction. Organic and inorganic polymers, both natural and synthetic, can be used as the insoluble support. Examples of suitable polymers include polyethylene, polypropylene, polybutylene, poly(4-methylbutylene), butyl rubber, silastic polymers, polyesters, polyamides, cellulose and cellulose derivatives (such as cellulose acetate, nitrocellulose and the like), acrylates, methacrylates, vinyl polymers (such as polyvinyl acetate, polyvinyl chloride, polyvinylidene chloride, polyvinyl fluoride, and the like), polystyrene and styrene graft copolymers, rayon, nylon, polyvinylbutyrate, polyformaldehyde, etc. Other materials which can be used as the insoluble support can the latexes of the above polymers, silica gel, silicon wafers, glass, paper, insoluble protein, metals, metalloids, metal oxides, magnetic materials, semi-conductive materials, cermets and the like. In addition are included substances which form gels, such as proteins such as gelatins, lipopolysaccharides, silicates, agarose, polyacrylamides or polymers which form several aqueous phases such as dextrans, polyalkylene glycols (alkyene with 2 to 3 carbon atoms) or surfactants, e.g. amphophilic ompounds such as phospholipids, long chain (12-24 carbon atoms) alkyl ammonium salts and the like.

A preferred diagnostic support of this invention comprises a polystyrene, styrene copolymers such as styrene-acrylonitrile copolymers, or polyolefins such as polyethylene or polypropylene, and acrylate and methacrylate polymers and copolymers. The anti-plaque reagent antibody or the plaque antigen can be bound to the insoluble support by adsorption, ionic bonding, van der Waals adsorption, electrostatic bonding, or other non-covalent bonding, or it can be bound to the insoluble support by covalent bonding. A particularly advantageous support for this procedure comprises a microtiter plate having a plurality of wells. The well surface or plastic cup inserts therein can constitute the antigen or antibody support. If the determination will require the use of fluorometric measurements, the microtiter plate or the well inserts are advantageously opaque to light so that excitation light applied to a well does not reach or influence contents of the surrounding wells.

Procedures for non-covalent bonding are described in U.S. Patent 4,528,267. Procedures for covalently bonding antibodies and antigens to insoluble supports are described by Ichiro Chibata in IMMOBILIZED ENZYMES. Halsted Press: New York (1978) and A. Cuatrecasas, J. Bio. Chem. 245:3059 (1970), the entire contents of which are hereby incorporated by reference. The surface can be coated with a protein and coupled with the antibody or antigen using procedures described in U.S. Patent 4,210,418 using glutaraldehyde as a coupling agent, for example. In a still further procedure, the well can be coated with a layer having free isocyanate groups such as a polyether isocyanate, and application of the antibody or antigen in aqueous solution thereto effects the requisite bonding. In a still further procedure, the antibody or antigen can be coupled to a hydroxylated material by means of cyanogen bromide as described in U.S. Patent 3,720,760.

Both labeled anti-plaque antibodies and anti-idiotype antibodies are useful in applications of the methods of this invention. The labeled anti-plaque antibodies and anti-idiotype antibodies can be prepared by conventional procedures for attaching labels to proteins. The labels can be bonded or coupled to the protein reagents by chemical or physical bonding. Ligands and groups which can be conjugated to the antibodies of this invention include elements, compounds or biological materials which have physical or chemical characteristics which can be used to distinguish the reagents to which they are bonded from compounds and materials in the sample being tested. As a general principle, methods suitable for linking a label with an antibody are equally suitable for linking or bonding the label to a plaque antigen. Although the following procedures are described in terms of conjugating labels to antibodies, their use to conjugate labels to plaque antigens are also intended.

Radiolabeled antibodies of this invention can be used for in vitro diagnostic tests. The specific activity of a tagged antibody depends upon the half-life, isotopic purity of the radioactive label and how the label is incorporated into the antigen or antibody. Table A lists several commonly used isotopes, their specific activities and half-lives. In immunoassay tests, the higher the specific activity, in general, the better the sensitivity.

## Table A

| Isotope | Specific Activity of Pure Isotope (Curies/mole) | Half-Life |
|---------|--------------------------------------------------|-----------|
| $^{14}C$ | 6.25 x 101 | 5720 years |
| $^{3}H$ | 2.91 x 104 | 12.5 years |
| $^{35}S$ | 1.5 x 106 | 87 days |
| $^{125}I$ | 2.18 x 106 | 60 days |
| $^{32}P$ | 3.16 x 106 | 14.3 days |
| $^{131}I$ | 1.62 x 107 | 8.1 days |

Procedures for labeling antibodies with radioactive isotopes listed in Table A are generally known in the art. Tritium labeling procedures are described in U.S. Patent 4,302,438, for example. Iodinating, tritium labeling and 35S labeling procedures especially adapted for antibodies are described by Goding, J.W. MONOCLONAL ANTIBODIES: PRINCIPLES AND PRACTICE. New York: Academic Press pp 124-126 (1983) and the references cited therein. Other procedures for iodinating antibodies are described by Hunter and Greenwood, Nature. 144:945 (1962) and David et al, Biochemistry. 13:1014-1021 (1974) and in U.S.Patents 3,867,517 and 4,376,110.

Radiolabeled antibodies useful for imaging and radiotherapy can also be used as reagents in the methods of this invention. Radiolabeling elements which are useful in imaging include $^{123}$I, $^{131}$I, $^{111}$In, and $^{99m}$Tc, for example. Procedures for iodinating antibodies are described by Greenwood, F. et al, " Biochem.J. 89:114-123 (1963); Marchalonis, J., Biochem.J. 113:299-305 (1969); and Morrison, M. et al, Immunochemistry. 8:289-297 (1971). Procedures for $^{99m}$Tc-labeling are described by Rhodes, B. et al, in TUMOR IMAGING: THE RADIOIMMUNOCHEMICAL DETECTION OF CANCER. Burchiel, S. et al (eds.), New York: Masson 111-123 (1982), and the references cited therein. Procedures suitable for $^{111}$In-labeling antibodies are described by Hnatowich, D.J. et al, J.Immul.Methods. 65:147-157 (1983); Hnatowich, D.J. et al, Science. 220:613-615 (1983) and Buckley, R.G. et al, F.E.B.S. 166:202-204 (1984).

Examples of suitable systems, coupling procedures and substrate reactions therewith are disclosed in U.S. Patents Re. 31,006, B1\3,654,090, 4,214,048, 4,289,747, 4,302,438, 4,312,943, 4,376,110 and the references cited therein, for example. Examples of other suitable systems are described by Pesce et al, Clin.Chem. 20(3):353-359 (1974) and Wisdom, G., Clin.Chem. 22:1243 (1976).

A list of suitable enzyme classes and specific examples for each class follow:

## TABLE I

| Class | Enzyme Example |
|-------|----------------|
| Hydrolases Carbohydroases | Amylases |
| Nucleases | Polynucleotidase |
| Amidases | Arginase |
| Purine deaminases | Adenase |
| Peptidases | Aminopolypeptidase |
| Proteinases | Pepsin |
| Esterases | Lipases |
| Iron Enzymes | Catalase |
| Copper Enzymes | Tyrosinases |
| Enzymes containing Coenzymes | Alcohol dehydrogenase |
| Enzymes reducing cytochrome | Succinic dehydrogenase |
| Yellow enzymes | Diaphorase |
| Mutases | Glyoxalase |
| Demolases | Aldolase |
| Oxidases | Glucose oxidase |
| | Horse radish |
| | peroxidase |
| Other enzymes | β-galactosidase |
| | Phosphatases |
| | Phosphorylases |
| | Hexokinases |

A list of suitable enzymes are described in Hawk, et al. PRACTICAL PHYSIOLOGICAL CHEMISTRY, New York: McGraw-Hill pp 306-397 (1954).

Fluorogenic enzymes (enzymes in the presence of which a selected substrate will produce a fluorescent product) are useful labeling moieties. Methods for selectively conjugating enzymes to antibodies without impairing the ability of the antibody to bind with antigen are well known in the art. Suitable enzymes and procedures for coupling them to antibodies are described by Ichiro Chibata in IMMOBILIZED ENZYMES (supra), A. Cuatrecasas, J. Bio. Chem. (supra), Wilson, M. et al, INTERNATIONAL CONFERENCE IN IMMUNOFLUORESCENCE AND RELATED STAINING TECHNIQUES. W. Knapp et al, editors. Amsterdam: Elsevier pp 215-244 (1978), Sullivan, M. et al, Annals of Clinical Biochemistry. 16:221-240 (1979), Nygren, H. et al, Medical Biology. 57:187-191 (1979), Gadkari, D. et al, Journal of Virological Methods. 10:215-224 (1985), Tijssen, P. et al, Analytical Biochemistry. 136:451-457 (1984), Tsuruta, J. et al, The Journal of Histochemistry and Cytochemistry. 33(8):767-777 (1985), and in U.S. Patent 4,190,496, for example, the entire contents of the above listed references being hereby incorporated by reference in their entireties. The preferred fluorogenic enzymes and suitable substrates corresponding thereto include horseradish peroxidase for which a suitable substrate is homovanillic acid or 4-hydroxy-3-methoxy-phenylacetic acid, β-galactosidase for which a suitable substrate is 4-methylumbelliferyl-β-D-galactoside, alkaline phosphatase for which a suitable substrate is 4-methylumbelliferyl phosphate and other umbelliferyl phosphates such as 4-carboxyumbelliferyl phosphate and umbelliferyl phosphate 4-carboxyalkyl-esters, etc.

Examples of suitable procedures for enzyme labeling the antibody include the use of carbodiimides, dialdehydes, and bifunctional coupling reagents. Linkage of enzymes through amide groups can be achieved by treating the proteins with thionyl chloride, N-hydroxysuccinimide or similar reagents in an anhydrous solvent such as dimethylformamide, dioxane, dimethylsulfoxide, tetrahydrofuran, or the like. Alternative coupling agents include carbodiimides such as 1-ethyl-3-(3-N,N'-dimethylaminopropyl)-carbodiimide or 1-cyclohexyl-3-(2-morpholinoethyl)carbodiimide methyl-p-toluenesulfonate.

The carbohydrate moiety of an enzyme can also be oxidized to an aldehyde and reacted with lysyl amino groups of immunoglobulins to form a Schiffs base. Reduction with sodium borohydride effects a stable linkage of enzyme and antibody. Horseradish peroxidase with antibody can be efficiently linked to immunoglobulins by the method of Wilson, supra.

Fluoroscent labeled antibodies can be prepared from standard fluorescent moieties known in the art. Since antibodies and other proteins absorb light having wavelengths up to about 310 nm, the fluorescent moieties should be selected to have substantial absorption at wavelengths above 310 nm and preferably above 400 nm. A variety of suitable fluorescers are described by Stryer, Science. 162:526 (1968) and Brand, L. et al, Annual Review of Biochemistry. 41:843-868 (1972). The antibodies can be labeled with fluorescent groups by conventional procedures such as those disclosed in U.S. Patents 3,940,475, 4,289,747 and 4,376,110, for example.

One group of fluorescers having a number of the desirable properties described above are the xanthene dyes, which include the fluoresceins derived from 3,6-dihydroxy-9-phenylxanthhydrol and resamines and rhodamines derived from 3,6-diamino-9-phenylxanthydrol and lissanime rhodamine B. The rhodamine and fluorescein derivatives of 9-o-carboxyphenylxanthhydrol have a 9-o-carboxyphenyl group. Fluorescein compounds having reactive coupling groups such as amino and isothiocyanate groups such as fluorescein isothiocyanate and fluorescamine are readily available.

Another group of fluorescent compounds are the naphthylamines, having an amino group in the alpha or beta position. Included among the naphthylamino compounds are 1-dimethylaminonaphthyl-5-sulfonate, 1-anilino-8-naphthalene sulfonate and 2-p-toluidinyl-6-naphthalene sulfonate. Other dyes include 3-phenyl-7-isocyanatocoumarin; acridines such as 9-isothiocyanatoacridine and acridine orange; N-[p-(2-benzoxazolyl)-phenyl]maleimide; benzoxadiozoles such as 4-chloro-7-nitrobenzo-2-oxa-1,3-diazole and 7-(p-methoxybenzylamino)-4-nitrobenzo-2-oxa-1,3-diazole; stilbenes such as 4-dimethylamino-4'-isothiocyanatostilbene and 4-dimethylanimo-4'-maleimidostilbene; N,N'-dioctadecycloxacarboxyamine-p-toluenesulfonate; pyrenes such as 8-hydroxy-1,3,6-pyrenetrisulfonic acid, 1-pyrenebutyric acid, merocyanine 540, rose bengal, 2,4-diphenyl-3(2H)-furanone, o-phthaldehyde, as well as other readily available fluorescing molecules. These dyes either have active functionalities or such functionalities can be readily introduced.

For example, antibodies can be labeled with fluorochromes by the procedures described by Goding, J. MONOCLONAL ANTIBODIES: PRINCIPLES AND PRACTICE. New York: Academic Press, pp 208-249 (1983). The concentration of fluorochrome is selected according to the table of Goding, p 229. For example, fluorescein isocyanate (1.0 mg/ml) or rhodamine isocyanate (10.0 mg/ml) in DMSO is prepared, and the desired volume (1-10% of total protein solution volume) is added to the protein solution dropwise, with stirring. The reaction proceeds for two hours, shielded from light. The product is purified by gel filtration on SEPHADEX G-25 gel in PBS containing 0.1% $NaNO_3$ to separate the unreacted or hydrolyzed fluorochrome. The absorbance of the conjugate is measured at 280 nm and at its peak in the visible region (495 nm for fluoresceinated antibody and 550 nm for rhodaminated antibody). The fluorochrome to protein ratio is calculated according to the procedure of Goding, supra, p 224-225. Conjugates are stored at 4°C protected from light until use. If the antibody solution concentration is less than 1 mg/ml, BSA is added to the solution to a final concentration of I mg/ml.

The antibodies of this invention can be covalently bonded to avidin or biotin in one embodiment of this invention. Suitable binding procedures involve cross-linking through a bifunctional cross-linking agent. Suitable bifunctional compounds are described by Peters, K. et al, Ann.Rev.Biochim. 46:523 (1977). Alkyl imidates show a high degree of specificity among the functional groups presented to them by a protein. The reaction is specific for primary amino groups. Examples of suitable coupling reagents include amidoesters such as dimethylmalonimidate, azides such as the acyl azide of tartryl diazide which reacts readily with immuno groups to produce amide linkages. Aryl dihalides (e.g., 1,5-difluoro-2,4-dinitrobenzene, or 4,4'-difluoro-3,3'-dinitrophenyl sulfone, glutaraldehyde, 1-ethyl-3-(3-dimethylaminopropyl)carbodiimide hydrochloride, dimaleinide, mixed anhydride, m-maleamidobenzoyl N-hydroxysucciinimide ester, and other known cross-linking agents.

The foregoing reagents provide essentially irreversible bonds. Bifunctional agents with functional groups such as disulfide or glycol may be used. These provide bonds which can be broken after the cross-linking reaction, if desired. Such reagents include dimethyl 3,3′-dithiobispropionimidate, succinimidyl-propionimidate, N-(3-fluoro-4,6-dinitrophenyl)-cystamine, tartryl diazide, tartryl di(glycylazide) and tartryl di-(epsilon-amino caproylazide).

In other instances, the bonds can be formed directly between the reagents themselves. For example, antibody can be bound to biotin through functional groups on the respective materials. As a specific example, biotin can be treated with periodate and reacted with antibody to give a Schiff base formation without inhibiting the biotin to avidin binding or blocking immunological activity of the antibody.

Known techniques using bifunctional cross-linking agents include the following: (a) a one-step glutaraldehyde linkage, Avrameas, S., Immunochemistry. 6:43 (1969); (b) two-step glutaraldehyde linkage, Avrameas, S., Immunochemistry. 8:1175 (1971); and (c) dimaleimide linkage, Kato, K. et al, Euro.J.Biochem. 62:285 (1966).

Antibodies can be labeled with metallic radionuclides according the procedure of Hnatowich, D. et al. Journal of Applied Radiation. 35(6):554-557 (1984) and Buckley, R et al. Federation of European Biochemical Societies. 166(1):202-204 (Jan. 1984). In this procedure the antibodies are conjugated with a chelating agent such as diethylenetriamine pentaacetic acid which is capable of forming a chelate with the metallic radionuclide. A suspension of 0.1 mg/ml of the bicyclic anhydride of DTPA (diethylenetriamine pentaacetic acid) is prepared in a dry solvent such as chloroform, ether or dry DMSO. An aliquot is removed to a clean, dry tube sufficient to provide a DTPA to immunoglobulin molar ratio of 1:1 and evaporated under nitrogen. A 10-20 microliter portion of the antibody solution used (10-20 mg/ml) in 0.05M bicarbonate buffer in saline, pH 7.0-7.5 is added to the dry DTPA, and the contents are agitated for 0.5-1.0 min. The coupled protein preparation is diluted to 0.2 ml with the same buffer solution and purifed on a 5 cm gel filtration column with SEPHADEX G-50 gel, using a saline eluant. The coupling efficiency is determined before purification by the addition of "chelation-grade" $^{111}$In in 0.5M acetate buffer solution, pH 6.0. Thin layer chromatograpy is used to separate the DTPA coupled antibody for calculation of the coupling efficiency. The DTPA-coupled antibodies can be stored at 4°C until needed for binding with metallic radionuclides such as $^{111}$In+3, $^{212}$Bi+3 and $^{68}$Ga+3, for example.

In the preferred sandwich immunoassay methods of this invention, an insoluble support to which preferably non-human anti-plaque antibody is adhered is contacted with patient serum for a sufficient time to permit conjugation of anti-idiotype antibody, if any, in the serum with the anti-plaque antibody on the insoluble support, and then removing the patient serum from the support. The incubation time should be sufficient to permit substantial conjugation to occur, the time being temperature dependent. Suitable incubation times are from 30 to 240 minutes at temperatures within the range of from 16 to 40°C, the preferred contact time being at least 60 minutes at temperatures within the range of from 20 to 26°C.

The residual serum is then removed form the support by use of a rinse solution. Any conventional rinse solution can be used. A preferred rinse solution is described in U.S.Patent 4,528,267. It is an aqueous phosphate buffer solution having a phosphate molarity of from 0.0001 to 0.05, a pH of from 6 to 8 and containing from 0.001 to 0.1 weight percent of non-ionic surfactant. Suitable non-ionic surfactants include polyoxyethylene ethers (BRIJ such as lauryl, cetyl, oleyl, stearyl, and tridecyl polyoxyethylene ethers; polyoxyethylene sorbitans (TWEEN such as polyoxyethylene sorbital monolaurate, monopalmitate, monostearate, monoleate, and trioleates); and other polyoxyethylene ethers (TRITON, for example). A preferred non-ionic surfactant is octylphenoxypolyethoxy ethanol having 40 ethylene oxide units (TRITON X-405, Rohm and Hass Company).

The insoluble support is then contacted with labeled non-human anti-human Ig antibody which will bind with any anti-idiotype antibody adhering to the insoluble support.

A variety of labels have been described above. For purposes of clarity and not by way of limitation, the subsequent steps of the process will be described for anti-human Ig antibodies which have been labeled with an enzyme, preferably a fluorogenic enzyme. The term "fluorogenic enzyme" is defined herein to refer to an enzyme which will produce a fluorophore product with a suitable substrate.

The enzyme labeled anti-human Ig antibody is applied to the insoluble support in an aqueous solution. The solution preferably contains suitable salts and buffers to preserve the reactants and facilitate the conjugation reaction. For example, the solution can contain bovine serum albumin (BSA), phosphate buffer solution (PBS), and a mild surfactant such as polyoxyethylene sorbitan ester employed in the above-described rinse solution. The incubation is continued for sufficient time to permit labeled anti-human Ig antibody to conjugate with serum anti-idiotype antibodies, if any, adhering to the insoluble support. The preferred incubation times and temperatures are as set forth for the conjugation of insolubilized anti-plaque antibody with the serum atherosclerotic anti-idiotype antibody.

The excess labeled anti-human Ig antibody solution is then removed form the insoluble support, and the support is rinsed with a rinse solution such as is describe above, to remove any residual, unconjugated labeled material.

In a third step of the sandwich process of this invention, the insoluble support is contacted with an aqueous solution of a substrate which undergoes a reaction in the presence of the enzyme to release fluorescent compound into the solution. Suitable substrates and the enzymes which which they can be converted are described in U.S.Patents 4,190,496 and 4,528,267, for example. The support is contacted with an aqueous solution of the substrate containing from $10^{-2}$ to $10^{-10}$ molar concentrations of the substrate. Substrate molar concentrations of from $10^{-4}$ to $10^{-5}$ are preferred. Preferred additional reagents and buffers in the substrate solution include 2-amino-2-methyl-1-propanol buffer and magnesium chloride, for example.

The substrate solution is incubated with the insoluble support for sufficient time for the reaction yielding the fluorophore to occur. At temperatures of from 18 to 40°C, incubation times of from 5 to 240 minutes can be used. Preferably, the temperature is within the range of from 20 to 26°C, and the incubation time is from 30 to 90 minutes.

The fluorescent level in the solution is then measured. The equipment and procedures for determining the level of fluorescence in the substrate solutions are those conventionally used in the art. The level of fluorescence is a function of the enzyme concentration on the insoluble support which is, in turn, a function of the amount of anti-idiotype antibody in the serum sample. The concentration of the atherosclerotic anti-idiotype antibody can be determined by comparing the fluorescence level of the solution with fluorescence levels obtained with control solutions containing known concentrations of anti-idiotype antibody.

In one competition immunoassay embodiment of this invention, an insoluble support having a known concentration of anti-idiotype antibody thereon is used. A mixture of the serum sample and a known concentration of labeled anti-plaque antibody is applied to the insoluble support for a time sufficient to permit anti-plaque antibody conjugation with anti-idiotype antibody to occur. The times and temperatures described above for anti-plaque antibody conjugation with anti-idiotype antibodies in the above-described sandwich immunoassay are suitable. After removing and rinsing the residual serum solution from the insoluble support, the label remaining on the insoluble support or in the serum solution mixture can be determined. If the label is a fluorogenic enzyme, the amount of label on the insoluble support can be determined by contacting the insoluble support with a suitable substrate solution, permitting the reaction yielding the fluorophore in the solution, and measuring the fluorescence level, as described above with respect to the sandwich immunoassay embodiment.

In another embodiment of a competition immunoassay, the insoluble support has anti-plaque antibody adhered thereto. A mixture of the serum sample and labeled anti-idiotype antibody is then incubated with the insoluble support for a time sufficient to permit anti-plaque antibody to conjugate with anti-plaque antibody. The amount of label on the insoluble support or remaining in serum solution is then determined. If the label is a fluorogenic enzyme, the amount of label on the insoluble support can be determined by contacting the insoluble support with a suitable substrate solution, permitting the reaction yielding the fluorophore in the solution, and measuring the fluorescence level, as described above with respect to the sandwich immunoassay embodiment.

This invention is further illustrated by the following specific, but non-limiting examples. Temperatures are given in degrees Centigrade and percents as weight percents unless otherwise specified. Examples which are constructively reduced to practice herein are presented in the present tense, and examples representing laboratory experiments previously reduced to practice are presented in the past tense.

EXAMPLE 1

Isolating Atherosclerotic Plaque Antigen

The intimal layer (plaque) is stripped from the medial layer, and washed briefly in 0.9 N Saline. The plaque is cut into small pieces (2 x 2 mm), and the tissue is homogenized in 0.15 M PBS at ph 7.2 at high speed for 30-60 sec at 4°C. The homogenate is centrifuged (2000 x g) at 4°C for 30 min. The supernatant is saved. The homogenizing and centrifuging steps are repeated, pooling the supernatants. Sodium azide in bacteriostatic quantities is added to the pooled supernatant, and the supernatant is stored at 4°C.

## EXAMPLE 2

Antibody conjugation to Sepharose

Freeze-dried CNBr-Sepharose 4B powder (Pharmacia) is swelled for 15 min in 1 mM HCl. The gel is washed on a sintered glass filter (porosity G-3) with a total of 200 ml of 1 mM HCl per gram of gel (dry wt.) This is done in several aliquots, the supernatant being suctioned off between successive additions.

5 mg of protein to be coupled per 1 ml of gel is dissolved in Coupling Buffer (0.1 M $NaHCO_3$, pH 8.0, containing 0.5 M NaCl). The gel is washed with Coupling Buffer, the excess is removed by suction, and the protein solution is mixed with the gel. The mixture is allowed to stand overnight at 4°C without stirring. The gel is then placed in a Blocking Buffer containing 1 M ethanolamine, pH 8.0, for 2 hr at rm temp. The gel is then washed three times with the Coupling Buffer. The protein-Sepharose conjugate is now ready for use and can be stored at 4 to 8°C. Cyanogen bromide can be added to the buffer solution as a bacteriostat.

## EXAMPLE 3

IgG Antibody Adsorption from Plaque Supernatant

A column is packed with 25 ml of Sepharose gel conjugated to anti-IgG antibody prepared in accordance with the procedure of Example 2, containing a total of about 130 mg of anti-IgG antibody. The column is equilibrated with from 2 to 3 volumes of buffer (0.15 M PBS, pH 7.2), and the sample is then applied to the column. The sample flow rate is 10-15 ml/min.

The column is then washed with 10 x volumes of 0.15 M PBS buffer, pH 7.2.

The column is then washed with sodium acetate buffer solution, pH 4.0 (Eluting Buffer) to desorb immunoaffinity bound IgG antibody. The column is eluted a rate of 15-20 ml/hr, collecting the eluted samples and retaining peak fractions. The peak fractions are dialyzed against 0.15 M PBS, pH 7.2, for 24-36 hr at 4°C with multiple buffer changes.

The column is then washed with 10 x volumes of 0.15 M PBS buffer, pH 7.2.

## EXAMPLE 4

IgE Antibody Adsorption from Plaque Supernatant

The procedure of Example 3 is repeated with a column packed with 7.5 ml of Sepharose gel conjugated to anti-IgE antibody prepared in accordance with the procedure of Example 2. The flow rate of Eluting Buffer is 15-20 ml/hr.

## EXAMPLE 5

IgA Antibody Adsorption from Plaque Supernatant

The procedure of Example 3 is repeated with a column packed with 7.5 ml of Sepharose gel conjugated to anti-IgA antibody prepared in accordance with the procedure of Example 2. The flow rate of Eluting Buffer is 15-20 ml/hr.

## EXAMPLE 6

IgM Antibody Adsorption from Plaque Supernatant

The procedure of Example 3 is repeated with a column packed with 10.0 ml of Sepharose gel conjugated to anti-IgM antibody prepared in accordance with the procedure of Example 2. The flow rate of Eluting Buffer is 15-20 ml/hr.

## EXAMPLE 7

Plaque Antigen Purification

IgG antibody binding specifically with atherosclerotic plaque obtained in accordance with the procedure of Example 3 is bound to Sepharose in accordance with the procedure of Example 2 to form an affinity column gel binding specifically to plaque antigen.

A column is packed with 25 ml of Sepharose gel conjugated to anti-plaque IgG antibody. The column is equilibrated with 2-3 volumes of buffer (0.15 M PBS, pH 7.2), and plaque solution obtained according to the procedure of Example 1 is applied to the column at a flow rate of 10-15 ml/min. The column is then washed 10 times with volumes of 0.15M PBS buffer, pH 7.2.

The flow rate of eluting buffer (0.1 M Sodium Acetate, pH 4.0) is 15-20 ml/hr. The eluted fractions containing plaque antigen are collected until peak activity disappears, yielding antigen to which the anti-plaque IgG antibody specifically binds.

The column is then washed with 10 x volumes of 0.15 M PBS buffer, pH 7.2.

Repeating this procedure but replacing the gel conjugated with anti-plaque IgG antibodies with gel conjugated with anti-plaque IgA, IgE and IgM antibodies obtained in accordance with the procedures of Examples 4-6, respectively, yields the corresponding plaque fractions to which the antibodies specifically bind.

## EXAMPLE 8

Polyclonal Anti-Plaque Antibodies

Polyclonal antiserum against atherosclerotic plaque antigen prepared in accordance with the procedure of Example 7 is elicited in rabbits using the immunization techniques and schedules described in the literature, e.g., Stollar, Methods of Enzymology. 70:70 (1980). The antiserum is screened in a solid phase assay similar to that used for monoclonal antibodies, e.g., as described by Lange et al, Clin.Exp.Immunol. 25:191 (1976) and Pisetsky et al, J.Immun.Methods. 41:187 (1981). The initial screening criterion would be binding to atherosclerotic plaque antigen.

The IgG fraction of the antisera is purified further by affinity chromatography on a column containing a resin on which the plaque antigen is immobilized, following the procedures of Example 3.

## EXAMPLE 9

Monoclonal Anti-Plaque Antibody

Using the purified atherosclerotic plaque antigen prepared in accordance with the procedures of Example 7, mouse monoclonal antibodies to the plaque antigen are obtained using standard procedures of Galfre and Milstein, Methods in Enzym. 73:1 (1981). The monoclonal antibodies are screened using a modification of the techniques described in the literature, e.g., Lange et al, Clin.Exp.Immunol. 25:191 (1976) and Pisetsky et al, J.Immun.Methods. 41:187 (1981). To be useful for the assay of serum plaque antigen (or immunocomplexes thereof), a monoclonal antibody should bind to the plaque antigen with high affinity (preferably, $K_A$ $10^{10}$ $M^{-1}$).

Mouse monoclonal antibody is purified in a two step procedure. The neat ascites fluid is applied to a column of Affi-Gel Blue resin (Bio-Rad Laboratories, Richmond, CA) equilibrated with 10 mM Tris-HCl, 0.15 M NaCl, pH 8.0, and eluted with the same buffer. This steps removes albumin, which is retained on the column. The final step in the purification is application to a DEAE-Sepharose (Pharmacia Fine Chemicals, Piscataway, NJ) and elution with a linear gradient of 10 mM Tris-HCl, pH 8.0, to 10 mM Tris-HCl, 100 mM NaCl. This gives purified mouse monoclonal antibody free from contaminating serum proteins such as albumin and transferrin.

EXAMPLE 10

Polyclonal Anti-idiotype Antibody

The procedure of Example 8 is repeated with the exception that the immunizing agent is human, anti-plaque antibody extracted from fresh, pooled human plaque prepared following the procedure of Example 3, and the antisera is purified further by affinity chromatography on a column containing a resin on which human anti-plaque antibody is immobilized following the procedure of Example 3. The eluate contains rabbit anti-idiotype antibody.·

EXAMPLE 11

Polyclonal Anti-idiotype Antibody

Polyclonal antiserum against atherosclerotic anti-plaque antibody prepared in accordance with the procedure of Example 8 or 9 is elicited in goats using as the immunizing agent, human anti-plaque antibody, and following the immunization techniques and schedules described in the literature, e.g., Stollar, Methods of Enzymology. 70:70 (1980). The antiserum is screened in a solid phase assay similar to that used for monoclonal antibodies, e.g., as described by Lange et al, Clin.Exp.Immunol. 25:191 (1976) and Pisetsky et al, J.Immun.Methods. 41:187 (1981). The initial screening criterion would be binding to atherosclerotic anti-plaque antibody.

The IgG fraction of the antisera is purified further by affinity chromatography on a column containing a resin on which the anti-plaque antibody is immobilized, following the procedures of Example 3.

EXAMPLE 12

Monoclonal Anti-idiotype antibodies

Following the procedure of Example 9 but screening the clones for supernatants binding to human anti-plaque antibody, hybridomas which yield anti-idiotype antibodies are obtained. Several clones producing different anti-idiotype antibodies are preferred for use of monoclonal antibody cocktails.

EXAMPLE 13

Monoclonal Anti-idiotype antibodies

Following the procedure of Example 9 but immunizing the mice with human polyclonal anti-plaque antibodies obtained by the procedures of Example 8 and screening the clones for supernatants binding to anti-plaque antibody, hybridomas which yield anti-idiotype antibodies are obtained. Several clones producing different anti-idiotype antibodies are preferred for use of monoclonal antibody cocktails.

EXAMPLE 14

Monoclonal Anti-idiotype antibodies

Following the procedure of Example 9 but immunizing the mice with rabbit or goat anti-plaque antibodies obtained by the procedures of Example 8 and screening the clones for supernatants binding to human anti-plaque antibody, hybridomas which yield anti-idiotype antibodies are obtained. Several clones producing different anti-idiotype antibodies are preferred for use of monoclonal antibody cocktails.

## EXAMPLE 15

Anti-plaque antibody Coated Micotiter Plate

100 Microliters of prepared dilutions of anti-plaque antibody prepared in accordance with the procedure of Example 8 are appled to the surface of ·IMMULON Il microtiter plates (Dynatech). The coating solution concentrations are selected to be from 1-5 micrograms/well but can be varied up or down depending upon the selection of other reagents and immunoassay procedures to be followed. The plates are tapped gently and to insure the coating solution covers the bottom of each well completely. The wells are incubated at 4°C overnight in a covered, humidified box.

The coating solution is discarded, and 200 microliters of 1% BSA in PBS is added per well. The wells are then incubated at rm temp for 1 hr in a humidity box, and the BSA solution is removed. The wells are then washed with 4 times with 200 microliters of Wash Buffer (PBS, 0.5% TWEEN, and 0.02% sodium azide), and stored in a humidity box at 4°C until use.

## EXAMPLE 16

Anti-idiotype Antibody Coated Microtiter Plate

Repeating the procedure of Example 15 but replacing the anti-plaque antibody with the anti-idiotype antibody produced in accordance with the procedure of Example 10 yields a microtiter plate with wells coated with polyclonal anti-idiotype antibody.

## EXAMPLE 17

Enzyme Labeled Anti-Plaque Antibody

Anti-plaque antibody prepared in accordance with the procedures of Example 8 or 9 is conjugated with alkaline phosphatase following the modified procedure of O'Sullivan, M. et al, Analytical Biochem. 100:100 (1979), the entire contents of which are hereby incorporated by reference.

Horseradish peroxidase is conjugated to anti-plaque antibody in accordance with the procedure of Nygren, H. et al, Medical Biology. 57:187-191 (1979) as follows. Horseradish peroxidase (HRP, Type II or Type VI, Sigma) is dissolved in 0.05 M carbonate: bicarbonate buffer, pH 9.5, containing 0.25% glutaraldehyde (GA, Polaron). After 2 hr at room temperature, the excess GA is separated from the GA-HRP on a Sephadex G-25 column (0.7 12 cm, Pharmacia) equilibrated with 0.15 M NaCl. The GA-HRP complex is, in a second step, mixed with the antibody in 0.05 M carbonate: bicarbonate buffer, pH 9.5, containing 0.15 M NaCl, at different IgG:HRP ratios for 16-64 hr at 4°C. The reaction is stopped by the addition of lysine to a final concentration of 0.02 M.

## EXAMPLE 18

Enzyme Labeled Anti-idiotype Antibody

Anti-idiotype antibody prepared in accordance with the procedures of Examples 10-14 are conjugated with alkaline phosphatase following the modified procedure of O'Sullivan, M. et al, Analytical Biochem. 100:100 (1979), the entire contents of which are hereby incorporated by reference.

Horseradish peroxidase is conjugated to anti-idiotype antibody in accordance with the procedure of Nygren, H. et al, Medical Biology. 57:187-191 (1979) as follows. Horseradish peroxidase (HRP, Type II or Type VI, Sigma) is dissolved in 0.05 M carbonate: bicarbonate buffer, pH 9.5, containing 0.25% glutaraldehyde (GA, Polaron). After 2 hr at room temperature, the excess GA is separated from the GA-HRP on a Sephadex G-25 column (0.7 12 cm, Pharmacia) equilibrated with 0.15 M NaCl. The GA-HRP complex is, in a second step, mixed with the antibody in 0.05 M carbonate: bicarbonate buffer, pH 9.5, containing 0.15 M NaCl, at different IgG:HRP ratios for 16-64 hr at 4°C. The reaction is stopped by the addition of lysine to a final concentration of 0.02 M.

EXAMPLE 19

Fluorescence Immunoassay Procedure for Atherosclerotic Anti-idiotype Antibodies

To each microtiter plate coated with a different concentration of anti-plaque antibodies prepared in accordance with the procedures of Example 10 is applied 100 microliters/well of the serum sample of the patient being tested, diluted 1:100. The plates are covered to prevent drying and incubated for 2 hr. The sera is removed, and the plate is washed 3 times with Wash Buffer.

100 Microliters/well of alkaline phosphatase conjugated anti-human Ig antibody (Sigma) is applied to each well, and the plates are covered to prevent drying and incubated 2 hr. The enzyme labeled antibody solution is removed, and the plates are washed 3 times with Wash Buffer.

100 Microliters/well of 4-methylumbelliferyl phosphate solution (3M Diagnostics Systems) is then applied ot the well. The microtiter plates are then read in a fluorometer (3M Diagnostics) every 10 minutes until the first 4096 reading or 1 hr is reached. The readings are continued every 30 min for another 2 hr.

EXAMPLE 20

Isolating Plaque Antigen

Human aortas are obtained at autopsy from patients who have died of myocardial infarction, cerebral vascular accident or from surgical procedures. The aortae are dissected free of surrounding tissue and fat and rinsed in saline to remove contaminating blood. Segments of plaques are separated and processed.

The plaque areas are removed and cut into small pieces of 2 x 2 mm. The tissue is washed briefly in saline and homogenized in isotonic phosphate-buffered saline (PBS) at pH 7.0-7.2 in a WARING BLENDER at high speed for 30 sec at 4°C. The homogenate is centrifuged (2000 x g) for 30 min at 4°C and eluted. The eluates from this and subsequent steps are saved and concentrated.

The sedimented debris is washed in cold PBS and spun repeatedly as above until the supernatant is clear and contains no chemically or immunochemically detectable protein. At his stage, over 95% of soluble protein have been removed. The sediment is then washed in distilled water and extracted with 0.02 M citrate buffer, pH 3.2 with constant stirring at 37°C for 2 hr. After this elution procedure, the mixture is centrifuged (2000 x g) for 30 min. The supernatant fraction is neutralized with 0.5 M NaOH and dialyzed overnight against PBS. The supernatant as well as the pooled PBS eluates are concentrated by vacuum dialysis and ultrafiltration to yield a concentrate of soluble plaque antigens.

For isolation of insoluble plaque antigens, portions of the aorta plaque are digested with different enzymes, the digest is extracted with PBS, and the eluates are concentrated as described above. The enzyme compositions used include collagenase [100 units/ml] in 0.05 M Tris buffer, pH 7.4 containing 5 mM CaCl; elastase [20 units/ml] in 0.035 M carbonate buffer, pH 8.8 containing 0.2% NaCl; DNase [500 units/ml] in 0.1 M monosodium phosphate buffer, pH 7.0 containing 2.5 mM $MgCl_2$; hyaluronidase [50 units/ml] in 0.1 M monosodium phosphate, pH 5.3 buffer containing 0.15 M NaCl; and neuraminidase [1 unit/ml] in modified Ringer's solution, pH 7.3 containing 2 mM EDTA. 10 Ml of digestion mixture is used per gram of aorta plaque. Each tissue-enzyme mixture is incubated at 37°C in a shaking water bath. The duration of incubation is 24 hr for collagenase and elastase, 15 hr for hyaluronidase and 1 hr for DNase and neuraminidase. Each digest is centrifuged (2000 x g), and the liquid portion is concentrated to 0.5-1.0 with AMICON ultrafiltration membranes (PM 10), dialyzed against 0.15 M PBS for 24 hours to yield a concentrated solution of insoluble plaque antigens.

EXAMPLE 21

Isolating Anti-Plaque Antibodies

A portion of the soluble extract obtained according to the procedure of Example 20 is processed to separate autoantibodies therefrom. The concentrate is diluted with 0.15 M sodium chloride to contain approximately 15 mg./ml of protein including anti-plaque antibodies. The proteins are precipitated with anhydrous sodium sulfate (18 g/100 ml) made at 25°C. After one hour, the precipitate is collected by centrifugation (10,000 x g, 20 min, 25°C) and dissolved in 0.8 volu. of 0.1 M PBS, pH 7.5. The material is reprecipitated again as described. The sediment is dissolved in 0.1 M tris-HCl buffer (pH 8.0, 20°C) and applied to a column (3.2 x 30 cm) of DEAE-SEPHADEX A-50 (Pharmacia) equilibrated with the same buffer. Elution of the material is performed by a continuous gradient from 0.1 M tris-HCl at a constant pH of 8.0 at 20°C. Fractions containing IgA, IgD, IgE, IgG and IgM immunoglobulins are collected and concentrated. They are then applied to a column (3.2 x 95 cm) of SEPHADEX G-150 (Pharmacia) equilibrated with 0.1 M tris-HCl, 0.2 M NaCl, 0.002 M EDTANa₂, pH 7.7 containing 0.2% sodium azide. The material was allowed to pass through the column three times (recycling chromatography technique). Fractions containing IgA, IgD, IgE, IgG and IgM immunoglobulins are collected and concentrated.

## EXAMPLE 22

Isolating Purified Plaque Antigens

The plaque antigens obtained according to the procedure of Example 20 is purified by affinity chromatography according to the procedure of Mishell and Shilgi in SELECTED METHODS IN CELLULAR IMMUNOLOGY. San Francisco: Freeman (1980), the entire contents of which are hereby incorporated by reference. A cross-linked agarose gel which has been substituted with isothiocyanatophenoxyhydroxypropyl groups is suspended in an aqueous solution of sodium bicarbonate, and to this suspension is added one ml of a mixed purified anti-plaque antibody solution obtained in accordance with the procedure of Example 2. The mixture is incubated for 3 days at room temperature while being gently stirred. The particles are then centrifuged and washed twice each of 0.5 M NaHCO₃, 0.1 M acetate buffer, and 0.1 M tris buffer containing 1 wt.% BSA.

The antibody bonded particles are mixed with a solution of plaque antigen concentrate prepared in accordance with Example 1 in PBS. The mixture is incubated for 8 hrs (overnight) at 25°C. The particles are then separated from the mixture and rinsed three times with PBS, pH 7.2, to remove residual unbound plaque antigen. The particles are then mixed with an excess of 2.5 M NASCN solution, pH 8.0, and incubated for 8 hrs at 25°C. The solution containing affinity purified plaque antigen is then concentrated by negative pressure dialysis or ultrafiltration, and the plaque antigen solution is stored at 4°C.

## EXAMPLE 23

Polyclonal Anti-Plaque Antibody

Rabbits are injected intramuscularly with a mixture of 0.5 mg of plaque antigen prepared by the procedure of Example 3 in 0.2 ml of 0.15 M sodium chloride solution and 0.8 ml of complete Freund's adjuvant. The immunization is repeated for 14 days and then each week for 3 weeks. After a further 10 days have passed, blood is removed from the rabbits, and antiserum is recovered from the blood by allowing it to coagulate and removing the clot.

## EXAMPLE 24

Radiolabeled Anti-idiotype Antibody

Anti-idiotype antibody prepared in accordance with the procedure of Example 10 is labeled with $^{125}$I by the procedure of Greenwood, F. et al, Biochem.J. 89:114-123 (1963).

A chloramine-T solution of 1.0 mg/ml in distilled water is prepared. 10 Micrograms of the anti-idiotype antibody is pipetted into a small tube on ice. Then 10 microliters of 0.1 M Tris-HCl, pH 8.0, is added to buffer the NaOH which is always present in the radioactive iodide to prevent oxidation to iodine. The desired volume of $^{125}$I is then added. From 1.0 to 10 microliters of the chloramine-T solution is added and mixed. Then 10 microliters of a saturated solution of tyrosine in water is added to terminate the reaction. Then is added 2.5 ml of PBS saline containing 0.1 % BSA and 10 mM NaN₃. The mixture is transferred to a disposable PD-10 Sephadex column (Pharmacia) and equilibrated with the same buffer. The sample is allowed to run in and the effluent is discarded. Then is added 3.5 ml of the same buffer. The effluent containing the iodinated anti-idiotype antibody is collected, free of unreacted iodide.

Repeating this procedure with the anti-idiotype antibodies prepared in accordance with the procedures of Examples 11-14 yields the respective anti-idiotype antibodies labeled with [125]I.

**Claims**

1. A method for determining the presence of atherosclerotic anti-idiotype antibodies in serum comprising

a) contacting the serum with anti-plaque antibody for a time sufficient to permit conjugation of the atherosclerotic anti-idiotype antibody with the anti-plaque antibody, and

b) determining atherosclerotic anti-idiotype antibody conjugated with the anti-plaque antibody.

2. The method of Claim 1 comprising

a) contacting the sample with an insoluble support to which non-human anti-plaque antibody is adhered for a time sufficient to permit anti-idiotype antibody conjugation with the anti-plaque antibody to occur, and removing residual sample from the support;

b) contacting the insoluble support from step (a) with labeled anti-human Ig antibody for sufficient time to permit conjugation of anti-human Ig antibody with atherosclerotic anti-idiotype antibody and removing unconjugated labeled anti-human Ig antibody from the support; and

c) determining the label present on the insoluble support.

3. The method of Claim 2 wherein the label is an enzyme and the label present on the insoluble support is determined by contacting the insoluble support with a substrate which in the presence of the enzyme, produces a detectable chromophore or fluorophore.

4. The method of Claim 3 wherein the enzyme is horseradish peroxidase.

5. The method of Claim 3 wherein the enzyme is alkaline phosphatase.

6. The method of Claim 1 comprising

a) contacting an insoluble support to which a predetermined amount of atherosclerotic anti-idiotype antibody is bound with a mixture of a predetermined amount of labeled anti-plaque antibody and serum for a time sufficient to permit conjugation between plaque anti-idiotype antibody and anti-plaque antibody to occur, and

b) determining the amount of labeled anti-plaque antibody bound to the insoluble support or remaining in the mixture.

7. The method of Claim 6 wherein the label is an enzyme and the label present on the insoluble support is determined by contacting the insoluble support with a substrate which in the presence of the enzyme, produces a detectable chromophore or fluorophore.

8. The method of Claim 7 wherein the enzyme is horseradish peroxidase.

9. The method of Claim 7 wherein the enzyme is alkaline phosphatase.

10. The method of Claim 1 comprising

a) contacting an insoluble support to which a predetermined amount of anti-plaque antibody is bound with a mixture of a predetermined amount of labeled atherosclerotic anti-idiotype antibody and serum for a time sufficient to permit conjugation between anti-idiotype antibody and anti-plaque antibody to occur, and

b) determining the amount of labeled atherosclerotic plaque anti-idiotype antibody bound to the insoluble support or remaining in the mixture.

11. The method of Claim 10 wherein the label is an enzyme and the label present on the insoluble support is determined by contacting the insoluble support with a substrate which in the presence of the enzyme, produces a detectable chromophore or fluorophore.

12. The method of Claim 11 wherein the enzyme is horseradish peroxidase.

13. The method of Claim 11 wherein the enzyme is alkaline phosphatase.

14. Atherosclerotic plaque anti-idiotype antibody.

15. The atherosclerotic plaque anti-idiotype antibody of Claim 14, wherein the antibody is a polyclonal antibody.

16. The atheroclerotic plaque anti-idiotype antibody of Claim 15, wherein the antibody is bonded to a distinctive label.

17. The atheroclerotic plaque anti-idiotype antibody of Claim 16, wherein the distinctive label is an enzyme label.

18. The atherosclerotic plaque anti-idiotype antibody of Claim 14, wherein the antibody is a monoclonal antibody.

19. The atheroclerotic plaque anti-idiotype antibody of Claim 18, wherein the antibody is bonded to a distinctive label.

20. The atheroclerotic plaque anti-idiotype antibody of Claim 19, wherein the distinctive label is an enzyme label.

21. The atherosclerotic plaque anti-idiotype antibody of Claim 19 wherein the distinctive label is a radioactive label.1